# EUROPÄISCHE PATENTSCHRIFT

(11) **EP 1 967 215 B1**
(45) Veröffentlichungstag und Bekanntmachung des Hinweises auf die Patenterteilung: **05.10.2011**
(21) Anmeldenummer: 08151612.2
(22) Anmeldetag: 19.02.2008
(51) Int. Cl.: A61L 9/04, A61B 19/00

(54) **Verfahren zur Reinigung und zur thermischen Desinfektion von Gegenständen**
Method for cleaning and thermal disinfection of objects
Procédé de nettoyage et de désinfection thermique d'objets

(30) Priorität: 20.02.2007 DE 102007009382
(43) Veröffentlichungstag der Anmeldung: 10.09.2008
(73) Patentinhaber: Melag oHG, 10829 Berlin (DE)
(72) Erfinder: Van der Waydbrink, Eberhard Dr., 14641, Paulinenaue (DE); Litzba, Guido, 12161, Berlin (DE)
(74) Vertreter: Gross, Felix

(56) Entgegenhaltungen:
- EP-A- 0 702 963
- EP-A- 1 454 637
- DE-A1- 4 342 573
- US-A1- 2004 118 432

## Beschreibung

Die Erfindung betrifft ein Verfahren zur Reinigung und zur thermischen Desinfektion von Gegenständen nach dem Oberbegriff des Anspruchs 1.

Aus der DE 43 42 573 C1 ist ein Verfahren zum Reinigen, Spülen und thermischen Desinfizieren von Labor- und Operationsinstrumenten bekannt. Dieses Verfahren umfasst mehrere, einzeln ausblendbare Reinigungsschritte, die unter Verwendung unterschiedlicher Wasserqualitäten durchgeführt werden. Wenn ein Vorspülen durchgeführt wird, wird die dazu verwendete Reinigungsflüssigkeit auf Temperaturen zwischen 45 und 60 °C erwärmt.

Aus der DE 44 40 774 A1 und der DE 196 25 253 A1 sind weitere Verfahren zum Reinigen, Spülen und thermischen Desinfizieren von medizinischen Geräten bzw. Motoren- und Antriebssystemen bekannt, bei denen ein ebenfalls ausblendbares Vorspülen unter Verwendung einer nicht aufgewärmten Spülflüssigkeit durchgeführt werden.

Aus der EP 1 454 637 A1 ist ein Verfahren zum Reinigen und Desinfizieren medizinischer Instrumente bekannt, bei dem kaltes, enthärtetes Wasser zum Vorspülen eingesetzt wird. Dies ist mit dem Nachteil verbunden, dass Klarspülerreste von vorhergehenden Reinigungsdurchläufen nur unter Schaumbildung entfernt werden können. Dadurch sinkt der Spüldruck während des Vorspülens, was in einer geringeren Reinigungsleistung während des Vorspülens resultiert, so dass beispielsweise Proteinablagerungen auf dem Spülgut nur unzureichend an- oder abgelöst werden können. Außerdem werden durch die Verwendung "kalten" Wassers keine definierten Bedingungen während des Vorspülens bereitgestellt.

Der vorliegenden Erfindung liegt das Problem zugrunde, ein Verfahren zur Reinigung und zur thermischen Desinfektion medizinischer Instrumente bereitzustellen, bei dem ein Vorreinigen unter definierten Bedingungen erfolgt und gleichzeitig für medizinische Instrumente geeignet ist, die mit Proteinen verunreinigt sind.

Dieses Problem wird mit einem Verfahren zur Reinigung zur thermischen Desinfektion von Gegenständen mit den Merkmalen des Anspruchs 1 gelöst. Ein solches Verfahren wird in einem Reinigungs-Desinfektionsgerät durchgeführt und umfasst die Schritte des Vorreinigens, des Hauptreinigens und des sich anschließenden Endspülens mit thermischer Desinfektion. Diese Schritte werden jeweils unter Verwendung einer Spülflüssigkeit durchgeführt, wobei Wasser oder mit einem Reinigungsmittel versetztes Wasser als Spülflüssigkeit besonders geeignet ist. Als Wasser kommt grundsätzlich enthärtetes oder voll entsalztes Wasser in Betracht. Die Temperatur der in den einzelnen Verfahrensschritten jeweils verwendeten Spülflüssigkeit ist während jedes Verfahrensschritts individuell bestimmbar, wozu das Reinigungs-Desinfektionsgerät, in dem das Verfahren durchgeführt wird, vorzugsweise einen entsprechenden Temperaturfühler als Messsonde aufweist.

Erfingdungsgemäß wird das Vorreinigen bei einer Temperatur von 25 bis 35 °C durchgeführt, wobei die Temperatur über einen Zeitraum von 1 Sekunde bis 20 Minuten konstant gehalten wird. Dies hat den Vorteil, dass stets unter reproduzierbaren Bedingungen gearbeitet wird, da sichergestellt wird, dass eine vorgegebene Temperatur auch tatsächlich über einen ebenso vorbestimmbaren Zeitraum eingehalten wird. Arbeitet man im Gegensatz dazu mit nicht aufgewärmter Spülflüssigkeit, so ergeben sich in Abhängigkeit der Temperatur der Spülflüssigkeit (diese kann beispielsweise je nach Jahreszeit bei Verwendung von Wasser aus einem gewöhnlichen Kaltwasseranschluss beträchtlich schwanken) erhebliche Schwankungen in der Verlässlichkeit des Reinigungsvorgangs und der Vergleichbarkeit verschiedener Reinigungsvorgänge miteinander. Reproduzierbare Verhältnisse ließen sich in diesem Fall nur schwer einstellen.

Das erfindungsgemäße Temperaturintervall von 25 bis 35 °C bietet den Vorteil, dass die meisten Proteine bei dieser Temperatur noch nicht koagulieren und sich somit nicht auf dem zu reinigenden Spül- bzw. Desinfektionsgut (das heißt beispielsweise auf den medizinischen Instrumenten) in nicht oder nur schwer zu entfernender Weise ablagern.

Die bei dem Temperaturintervall des Vorreinigens angegebene untere Grenze ist derart gewählt, dass Lipide noch eine Mindestlöslichkeit in der zum Vorreinigen eingesetzten Spülflüssigkeit aufweisen, damit Zellen und andere lipidhaltigen Verunreinigungen, leichter bzw. überhaupt von dem Spül- bzw. Desinfektionsgut gelöst werden können und damit folglich ein intensiveres Vorspülen stattfindet als dies bei einer Spülflüssigkeit mit deutlich niedrigerer Temperatur der Fall ist. Das erfindungsgemäße Durchführen des Vorreinigens in dem angegebenen Temperaturintervall ermöglicht es, Gewebe (welches regelmäßig zahlreiche Lipide enthält) und darin angereichertes Blut (welches zahlreiche Proteine enthält) gleichermaßen effektiv von dem zu reinigenden und desinfizierenden Spül- bzw. Desinfektionsgut zu entfernen bzw. auf diesem für eine spätere Entfernung anzulösen.

Ein weiterer Vorteil des erfindungsgemäßen Verfahrens ist, dass das Hauptreinigen und das Endspülen mit enthärtetem Wasser oder mit voll entsalzendem Wasser durchgeführt werden. Dabei kann die Wasserqualität (das heißt entweder voll entsalztes oder enthärtetes Wasser) in beiden Schritten gleich oder unterschiedlich sein. Für empfindliches Spülgut ist es vorteilhaft, wenn sowohl im Hauptreinigen als auch im Endspülen voll entsalztes Wasser verwendet wird, wodurch jedoch verhältnismäßig hohe Kosten entstehen. Bei zahlreichen Anwendungen kann es von Vorteil sein, nur das Endspülen mit thermischer Desinfektion mit voll entsalztem Wasser durchzuführen, während das Hauptreinigen mit enthärtetem Wasser durchgeführt wird. Es ist zwar grundsätzlich möglich, auch das Endspülen mit thermischer Desinfektion mit enthärtetem Wasser durchzuführen, eine Durchführung mit voll entsalztem Wasser wird aus Gründen der Minimierung der Rückstände auf dem Reinigungsgut in zahlreichen Anwendungsfällen bevorzugt werden.

Das Vorreinigen findet erfindungsgemäß ausschließlich mit enthärtetem Wasser statt, wodurch Kosten gespart werden und aufwändig herzustellendes voll entsalztes Wasser nicht unnötig verbraucht wird. Sich gegebenenfalls auf dem Reinigungsgut ablagernde Verunreinigungen, die im enthärteten Wasser enthalten sein könnten, werden in den nachfolgenden Reinigungs- und Spülschritten entfernt. Die Menge solcher Ablagerungen hängt jedoch davon ab, welche Qualität das enthärtete Wasser aufweist. So gibt es enthärtetes Wasser, das nur einen sehr geringen Gehalt an gelösten Salzen aufweist, wodurch die Gefahr der Entstehung von Ablagerungen signifikant vermindert wird.

Als voll entsalztes Wasser (VE-Wasser) im Sinne der vorliegenden Erfindung wird Wasser mit einer Leitfähigkeit von nicht mehr als 15 µS/cm verstanden. Solchermaßen voll entsalztes Wasser weist eine Gesamthärte von nicht mehr als 0,02 mmol CaO pro Liter auf. Als enthärtetes Wasser im Sinne der vorliegenden Erfindung wird Wasser mit einer Gesamthärte von nicht mehr als 0,5 mmol CaO pro Liter verstanden. Dies entspricht einer Gesamthärte von nicht mehr als 3° dH. Diese Angaben sind der "Leitlinie von DGKH, DGSV und AKI für die Validierung und Routineüberwachung maschineller Reinigungs- und Desinfektionsprozesse für thermostabile Medizinprodukte und zu Grundsätzen der Geräteauswahl (Stand August 2006)", insbesondere den Seiten 31 und 56, entnommen.

In Abhängigkeit vom harten Ausgangswasser, das zur Enthärtung verwendet wird, kann enthärtetes Wasser mit den angegebenen Spezifikationen unterschiedliche Leitfähigkeiten aufweisen, da die Leitfähigkeit von enthärtetem Wasser und nicht enthärtetem Wasser infolge dessen, dass bei der Enthärtung lediglich Calcium- und Magnesiumionen gegen Natriumionen ausgetauscht werden, prinzipiell gleich ist. Leitungswasser, welches eine Härte von nicht mehr als 3° dH aufweist, wird auch als enthärtetes Wasser im Sinne dieser Erfindung verstanden, obwohl kein Enthärtungsschritt zur Bereitstellung dieses Wassers notwendig ist. Derart weiches Leitungswasser ist zwar selten, wird in Deutschland jedoch unter anderem in Ilmenau (Ortsteil Manebach, Gesamthärte: 1,8° dH) und Röttersdorf (Gesamthärte: 2,8° dH) bereitgestellt.

Die zu reinigenden und zu desinfizierenden Gegenstände, das heißt das Reinigungs- bzw. Desinfektionsgut, sind vorzugsweise medizinische Instrumente.

In einer bevorzugten Ausgestaltung der Erfindung umfasst das Verfahren zusätzlich ein ein-oder mehrfaches Zwischenspülen, ein ein- oder mehrfaches Neutralisieren und/oder ein ein-oder mehrfaches Trocknen. Der Zwischenspülschritt oder die Zwischenspülschritte können dabei zwischen dem Vorreinigen und dem Hauptreinigen und/oder zwischen dem Hauptreinigen und dem Endspülen durchgeführt werden. Der Schritt des Neutralisierens wird vorzugsweise nach dem Hauptreinigen durchgeführt, wenn zum Hauptreinigen ein alkalischer Reiniger verwendet wird. In diesem Fall wird während des Neutralisierens eine Spülflüssigkeit verwendet, die einen pH-Wert aufweist, der kleiner als 7 ist. Hierzu bieten sich wässrige Lösungen üblicherweise verwendeter Säuren an. Es ist ebenso denkbar, dass das Hauptreinigen bei einem neutralen pH-Wert durchgeführt wird, was insbesondere dann der Fall ist, wenn ein enzymatischer Reiniger während des Hauptreinigens eingesetzt wird. In diesem Fall ist der zusätzliche Schritt des Neutralisierens nicht notwendig.

Der Schritt oder die Schritte des Trocknens werden in einer bevorzugten Ausgestaltung der Erfindung als Gebläsetrocknen durchgeführt. Dies stellt eine besonders effiziente Trocknungsweise dar.

Zur Reduzierung des Bedarfs an voll entsalztem Wasser wird das Zwischenspülen vorzugsweise ausschließlich mit enthärtetem Wasser durchgeführt.

Für den Schritt des Neutralisierens wird bevorzugterweise enthärtetes Wasser oder voll entsalztes Wasser verwendet. Die Auswahl der zu verwendenden Wasserqualität richtet sich vorzugsweise danach, wie viele Reinigungs- oder Spülschritte auf das Neutralisieren folgen werden bzw. wie stark der pH-Wert der Neutralisierungslösung vom neutralen pH abweicht. Je größer der pH-Wert der Spülflüssigkeit, die zum Neutralisieren eingesetzt wird, vom neutralen pH-Wert abweicht, desto eher kann mit enthärtetem Wasser gearbeitet werden, da aufgrund der Menge der durch den Neutralisierungsprozess entstehenden Salze mit höherer Wahrscheinlichkeit ein weiterer Zwischenspülgang durchgeführt werden muss. Sind sowieso noch weitere Zwischenspülschritte vorgesehen, kann das Neutralisieren ebenfalls unter Verwendung von enthärtetem Wasser durchgeführt werden.

Zur Sicherstellung des Erreichens eines vorgegebenen pH-Wertes beim Hauptreinigen kann es aufgrund der Säurebindungskapazität von enthärtetem Wasser nötig sein, vorzugsweise voll entsalztes Wasser einzusetzen. Die Säurebindungskapazität von Wasser ist dessen Puffervermögen bezüglich des pH-Wertes, das durch Hydrogencarbonationen bestimmt wird. Dieses Puffervermögen ist unabhängig davon, ob Calcium- oder Magnesiumionen (bei nicht enthärtetem Wasser) oder z. B. Natriumionen (bei enthärtetem Wasser) als Gegenionen vorhanden sind.

In dem Fall, in dem beim Neutralisieren der pH-Wert der Spülflüssigkeit nur gering angepasst werden muss und die Anzahl der nachfolgenden Zwischenspülschritte gering gehalten werden soll, bietet sich ebenfalls eine Verwendung von voll entsalztem Wasser zum Neutralisieren an, um zusätzliche nachfolgende Zwischenspülschritte einzusparen.

Um gute Trocknungsergebnisse zu erzielen, also ein möglichst gut getrocknetes Spül- bzw. Desinfektionsgut am Ende des erfindungsgemäßen Verfahrens zu erhalten, wird das Trocknen vorzugsweise bei einer Temperatur von 40 bis 110 °C durchgeführt. Die untere Grenze dieses Temperaturintervalls kann auch bei 45, 50, 55, 60, 65, 70, 75, 80, 85, 90, 95 oder 100 °C angesetzt werden. Die obere Grenze des Temperaturintervalls ist auch mit 70, 75, 80, 85, 90, 95, 100 oder 105 °C ansetzbar. Die zuvor genannten oberen und unteren Grenzen sind im Rahmen der Bildung eines mathematisch sinnvollen Temperaturintervalls beliebig miteinander kombinierbar.

Um für eine gute Spül- bzw. Neutralisierungsleistung zu sorgen, werden das Zwischenspülen und/oder das Neutralisieren in einer bevorzugten Ausgestaltung der Erfindung unter Zufuhr von Wärmeenergie durchgeführt. Dabei ist es nicht notwendig, eine bestimmte, zu erreichende Temperatur vorzugeben. Vielmehr kann undefiniert geheizt werden, um eine insgesamt erhöhte Temperatur der für die Schritte des Zwischenspülens und/oder des Neutralisierens eingesetzten Spülflüssigkeiten zu erreichen. Auf diese Weise wird erreicht, dass bereits erwärmtes Spül- bzw. Desinfektionsgut nicht unnötig abgekühlt wird und umgekehrt noch nicht erwärmtes Spül- bzw. Desinfektionsgut vor Durchführung des Hauptreinigens bzw. des Endspülens bereits erwärmt wird, um so die entsprechende Aufheizung während des Hauptreinigens bzw. des Endspülens zu beschleunigen. Die zuvor dargestellte Durchführung des Zwischenspülen und/oder des Neutralisierens unter Zufuhr von Wärmeenergie kann auch als Umwälzen unter Heizen ohne Zieltemperatur bezeichnet werden.

Vorzugsweise ist die Dauer des Zwischenspülens und/oder des Neutralisierens vorgebbar, das heißt, dass diese Schritte unabhängig von der Temperatur während eines bestimmten Zeitintervalls durchgeführt werden.

In einer weiteren bevorzugten Ausgestaltung der Erfindung werden das Zwischenspülen und/oder das Neutralisieren bei einer vorgebbaren Temperatur durchgeführt. Das heißt, dass in diesem Fall nicht die Zeit, sondern die Temperatur der entscheidende Einflussfaktor auf die Schritte des Zwischenspülens und/oder des Neutralisierens ist.

Vorzugsweise wird die Temperatur während des Trocknens, des Zwischenspülens und/oder des Neutralisierens bzw. eines weiteren Schritts, in dem die Temperatur vorgegeben wird, während eines Zeitraums von 1 Minute bis 60 Minuten konstant gehalten. Die untere Grenze dieses Zeitraums kann auch bei 2, 3, 4, 5, 10 oder 15 Minuten liegen. Ebenso kann die obere Grenze dieses Zeitraums bei 5, 6, 7, 8, 9, 10, 15, 20, 30, 40 oder 50 Minuten liegen. Entsprechende Zeiträume sind im Rahmen dieser Erfindung durch wahlweise Kombination der angegebenen unteren und oberen Grenzen beliebig definierbar.

Durch das konstante Halten der Temperatur während eines vorgebbaren Zeitraums kann die Reproduzierbarkeit des Verfahrens weiter erhöht werden. Wird hingegen ein Verfahrensschritt nur in einem vorgebbaren Zeitraum ohne Temperaturkontrolle durchgeführt, können sich in Abhängigkeit der tatsächlichen Temperatur der Spülflüssigkeit erhebliche Unterschiede in der Reinigungs- oder Spülwirkung ergeben. Wird jedoch sichergestellt, dass die Spülflüssigkeit während eines vorgebbaren Zeitraums eine bestimmte, auswählbare Temperatur aufweist, wird eine Mindestreinigungsleistung bzw. Mindestspülleistung in dem betreffenden Verfahrensschritt nicht unterschritten.

In einer alternativen Ausgestaltung der Erfindung ist es vorgesehen, dass das ein- oder mehrfache Zwischenspülen, Neutralisieren und/oder Trocknen jeweils zumindest bis zum Erreichen einer vorgebbaren Temperatur der Spülflüssigkeit durchgeführt werden. Diese Durchführungsvariante entspricht im Wesentlichen der Durchführung eines Verfahrensschritts bei einer bestimmten vorgebbaren Temperatur über einen definierten Zeitraum, nur dass in diesem Fall die Temperatur lediglich einmal erreicht werden muss, nicht jedoch während eines nachfolgenden Zeitintervalls tatsächlich gehalten werden muss, um den entsprechenden Verfahrensschritt als abgeschlossen gelten zu lassen. Sofern diese Vorgehensweise mit der erwünschten Reinigungs-, Spül- oder Trocknungswirkung zu vereinbaren ist, lässt sich das entsprechende Verfahren auf diese Weise abkürzen.

In einer weiteren bevorzugten Variante des erfindungsgemäßen Verfahrens wird neben der Temperatur mindestens noch ein weiterer physikalischer Parameter der in den jeweiligen Verfahrensschritten eingesetzten Spülflüssigkeit bestimmt. Als entsprechende physikalische Parameter sind insbesondere der pH-Wert und/oder der Leitwert der Spülflüssigkeit vorgesehen. Der elektrische Leitwert G (der auch als Wirkleitwert oder Konduktanz bezeichnet wird) ist dabei der Kehrwert des elektrischen Widerstands R. Die Bestimmung des entsprechenden Parameters kann beispielsweise durch das Vorsehen einer entsprechenden Messanordnung (beispielsweise in Form einer Messsonde) in dem Reinigungs-Desinfektionsgerät, in dem das erfindungsgemäße Verfahren durchgeführt wird, bewerkstelligt werden.

In einer vorteilhaften Ausgestaltung der Erfindung, wird insbesondere der Leitwert, der während des Endspülens mit thermischer Desinfektion einzusetzenden Spülflüssigkeit bestimmt. Übersteigt dieser Leitwert einen bestimmten vorgebbaren Leitwert, wird die Spülflüssigkeit ausgetauscht, ohne dass zuvor eine thermische Desinfektion durchgeführt wird. Ein erhöhter Leitwert ist ein Indiz für Reste von Verunreinigungen in der Spülflüssigkeit, die beispielsweise durch Rückstände des im Hauptreinigen eingesetzten Reinigungsmittels oder andere (chemische) Rückstände verursacht werden. Das Vorhandensein solcher Verunreinigungen bzw. Rückstände während der Desinfektionsphase ist jedoch unerwünscht, da unvorteilhafte Reaktionen der Rückstände bei erhöhter Temperatur bzw. unerwünschte Ablagerungen auf dem Spül- bzw. Desinfektionsgut die Folge sein könnten.

Nach Austausch der Spülflüssigkeit im Endspülschritt wird der Leitwert vorzugsweise erneut gemessen. Übersteigt er abermals einen vorgebbaren Leitwert, wird die Spülflüssigkeit abermals ausgetauscht. Bleibt der Leitwert jedoch unter dem vorgegebenen Leitwert, kann der Schritt des Endspülens mit thermischer Desinfektion wie geplant vollständig durch Einleiten der thermischen Desinfektion durchgeführt und abgeschlossen werden.

Weitere Vorteile und Einzelheiten der Erfindung sollen anhand eines Ausführungsbeispiels im Zusammenhang mit einer Zeichnung näher erläutert werden. Es zeigt:

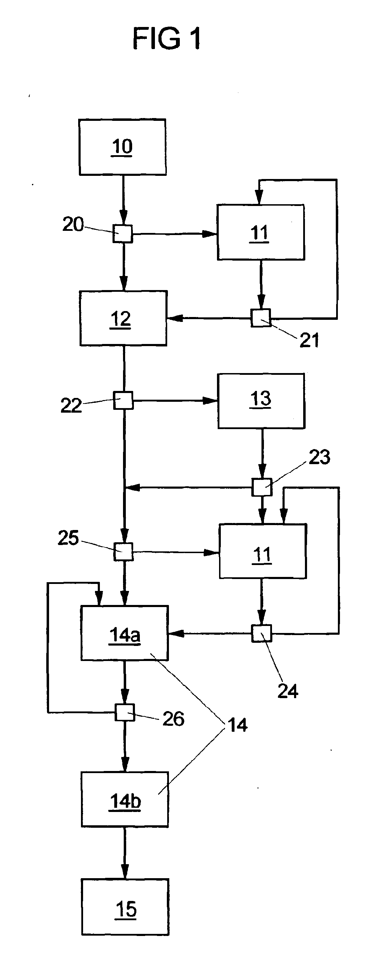

- Fig.1 1: ein schematisches Ablaufdiagramm eines Ausführungsbeispiels des erfindungsgemäßen Verfahrens.

Die Fig. 1 zeigt anhand eines Flussdiagramms in schematischer Weise den Ablauf eines Ausführungsbeispiels eines erfindungsgemäßen Verfahrens.

Ein Vorreinigen 10 stellt den ersten Verfahrensschritt dar. Nach Abschluss des Vorreinigens 10 kann an einem ersten Entscheidungspunkt 20 entschieden werden, ob ein Zwischenspülen 11 durchgeführt wird oder nicht. Wenn ein Zwischenspülen 11 durchgeführt wird, kann nach Ende dieses Zwischenspülschritts 11 an einem zweiten Entscheidungspunkt 21 entschieden werden, ob der Zwischenspülschritt 11 wiederholt werden soll oder nicht. Falls der Zwischenspülschritt 11 nicht wiederholt wird, läuft das Verfahren mit dem Schritt des Hauptreinigens 12 weiter. Das Hauptreinigen 12 ist dabei der Verfahrensschritt, der unmittelbar auf das Vorreinigen folgt, wenn am ersten Entscheidungspunkt 20 entschieden wird, dass kein Zwischenspülen 11 durchgeführt werden soll.

Nach erfolgreichem Abschluss des Hauptreinigens 12 kann an einem dritten Entscheidungspunkt 22 entschieden werden, ob ein Neutralisieren 13 durchgeführt werden soll oder nicht. Ein Neutralisieren 13 ist in der Regel nur dann erforderlich, wenn beim Hauptreinigen 12 ein Reinigungsmittel verwendet wurde, dessen pH-Wert von einem neutralen pH-Wert abweicht.

Das Neutralisieren 13 wird in der Regel nur einmal durchgeführt, kann jedoch auch mehrmals durchgeführt werden. Nach Abschluss des Neutralisierens 13 kann an einem vierten Entscheidungspunkt 23 entschieden werden, ob ein Zwischenspülschritt 11 durchgeführt werden soll oder nicht. Falls ein Zwischenspülschritt 11 durchgeführt wird, kann an einem fünften Entscheidungspunkt 24 entschieden werden, ob der Schritt des Zwischenspülens 11 wiederholt werden soll oder nicht.

Für den Fall, dass am vierten Entscheidungspunkt 23 entschieden wurde, dass kein Zwischenspülschritt 11 durchgeführt wird, folgt auf das Neutralisieren 13 das Endspülen mit thermischer Desinfektion 14, das sich in die Unterschritte des Endspülens 14a und der thermischen Desinfektion 14b untergliedert. Das Endspülen mit thermischer Desinfektion 14 folgt auch dann auf den Zwischenspülschritt 11, wenn am fünften Entscheidungspunkt 24 entschieden wird, dass das Zwischenspülen 11 nicht erneut durchgeführt wird.

Das Endspülen mit thermischer Desinfektion 14 kann sich auch unmittelbar an das Hauptreinigen 12 anschließen, wenn am dritten Entscheidungspunkt 22 entschieden wurde, keinen Neutralisierungsschritt 13 durchzuführen, und an einem sechsten Entscheidungspunkt 25 entschieden wurde, keinen Zwischenspülschritt 11 durchzuführen.

In einer alternativen Ausgestaltung des Verfahrens ist es ebenso möglich, dass am sechsten Entscheidungspunkt 25, der zwischen dem dritten Entscheidungspunkt 22 und dem Endspülen 14a angeordnet ist, einen oder mehrere Zwischenspülschritte 11 durchzuführen, ohne zuvor einen auf das Hauptreinigen 12 folgenden Neutralisierungsschritt 13 durchführen zu müssen.

Nach dem Endspülen 14a kann an einem siebten Entscheidungspunkt 26 vor dem Unterschritt der thermischen Desinfektion 14b entschieden werden, ob das Endspülen 14a erneut durchgeführt werden soll. Diese Entscheidung kann vorzugsweise in Abhängigkeit des Leitwerts der Spülflüssigkeit, mit der das erfindungsgemäße Verfahren durchgeführt wird, erfolgen. Übersteigt der Leitwert der Spülflüssigkeit einen vorgebbaren Grenzwert, ist dies ein Indiz dafür, dass noch Restchemikalien (insbesondere von den eingesetzten Reinigungsmitteln stammend) in der Spülflotte bzw. der Spülflüssigkeit enthalten sind. Da dies unerwünscht ist, wird in einem solchen Fall das Endspülen 14a erneut durchgeführt, bevor sich daran der Unterschritt der thermischen Desinfektion 14b anschließt.

Die in der Fig. 1 gesondert dargestellten Schritte des Endspülens 14a und der thermischen Desinfektion 14b stellen normalerweise einen gemeinsamen Verfahrensschritt des Endspülens mit thermischer Desinfektion 14 dar.

Nach erfolgreichem Abschluss des Endspülens mit thermischer Desinfektion 14 wird das Reinigungs- bzw. Desinfektionsgut in einem Trocknungsschritt 15, der vorzugsweise als Gebläsetrocknen ausgeführt wird, getrocknet. Nach erfolgreichen Abschluss dieses Trocknens 15 können die gereinigten, desinfizierten und getrockneten Gegenstände aus einem Reinigungs-Desinfektionsgerät, in welchem das erfindungsgemäße Verfahren durchgeführt wird, entnommen werden.

Wahlweise kann auch auf den Schritt des Trocknens 15 verzichtet werden, wenn die Trocknung der zu reinigen bzw. zu desinfizierenden Gegenstände entbehrlich erscheint.

### Bezugszeichenliste

- 10: Vorreinigen
- 11: Zwischenspülen
- 12: Hauptreinigen
- 13: Neutralisieren
- 14a: Endspülen
- 14b: thermische Desinfektion
- 14: Endspülen mit thermischer Desinfektion
- 15: Trocknen
- 20: erster Entscheidungspunkt
- 21: zweiter Entscheidungspunkt
- 22: dritter Entscheidungspunkt
- 23: vierter Entscheidungspunkt
- 24: fünfter Entscheidungspunkt
- 25: sechster Entscheidungspunkt
- 26: siebter Entscheidungspunkt

## Patentansprüche

1. Verfahren zur Reinigung und zur thermischen Desinfektion von Gegenständen in einem Reinigungs-Desinfektionsgerät, mit den folgenden Schritten:
• Vorreinigen (10),
• Hauptreinigen (12) und
• Endspülen mit thermischer Desinfektion (14),
wobei die Temperatur einer jeweils verwendeten Spülflüssigkeit während jedes Schritts (10, 12, 14) bestimmbar ist, und wobei
• das Hauptreinigen (12) und das Endspülen (14a) mit enthärtetem Wasser oder mit voll entsalztem Wasser durchgeführt werden und
• das Vorreinigen (10) ausschließlich mit enthärtetem Wasser durchgeführt wird,
**dadurch gekennzeichnet,**
**dass** das Vorreinigen (10) bei einer Temperatur von 25 bis 35 °C durchgeführt wird, wobei die Temperatur in einem Zeitraum von 1 s bis 20 min konstant gehalten wird.

2. Verfahren nach Anspruch 1, **dadurch gekennzeichnet, dass** es zusätzlich mindestens einen der nachfolgenden Schritte umfasst:
• Zwischenspülen (11),
• Neutralisieren (13) oder
• Trocknen (15),
wobei jeder zusätzliche Schritt auch mehrfach ausgeführt werden kann.

3. Verfahren nach Anspruch 2, **dadurch gekennzeichnet, dass** das Zwischenspülen (11) ausschließlich mit enthärtetem Wasser durchgeführt wird.

4. Verfahren nach Anspruch 2 oder 3, **dadurch gekennzeichnet, dass** das Neutralisieren (13) mit enthärtetem Wasser oder mit voll entsalztem Wasser durchgeführt wird.

5. Verfahren nach einem der Ansprüche 2 bis 4, **dadurch gekennzeichnet, dass** das Trocknen (15) bei einer Temperatur von 40 bis 110 °C durchgeführt wird.

6. Verfahren nach einem der Ansprüche 2 bis 5, **dadurch gekennzeichnet, dass** das Zwischenspülen (11) und/oder das Neutralisieren (13) unter Zufuhr von Wärmeenergie durchgeführt werden.

7. Verfahren nach Anspruch 6, **dadurch gekennzeichnet, dass** die Dauer des Zwischenspülens (11) und/oder des Neutralisierens (13) vorgebbar ist.

8. Verfahren nach Anspruch 6 oder 7, **dadurch gekennzeichnet, dass** das Zwischenspülen (11) und/oder das Neutralisieren (13) bei einer vorgebbaren Temperatur durchgeführt werden.

9. Verfahren nach Anspruch 5 oder 8, **dadurch gekennzeichnet, dass** die Temperatur während des Trocknens, des Zwischenspülens und/oder des Neutralisierens während eines Zeitraums von 1 min bis 60 min konstant gehalten wird.

10. Verfahren nach einem der Ansprüche 2 bis 9, **dadurch gekennzeichnet, dass** der Schritt oder die Schritte des Zwischenspülens (11), des Neutralisierens (13) und/oder des Trocknens (15) jeweils zumindest bis zum Erreichen einer vorgebbaren Temperatur andauern.

11. Verfahren nach einem der vorhergehenden Ansprüche, **dadurch gekennzeichnet, dass** neben der Temperatur mindestens ein weiterer physikalischer Parameter, insbesondere der pH-Wert und/oder der Leitwert, der Spülflüssigkeit während jedes Schritts bestimmbar ist.

12. Verfahren nach Anspruch 11, **dadurch gekennzeichnet, dass** die Spülflüssigkeit zu Beginn des Endspülens (14a) vor der thermischen Desinfektion (14b) ausgetauscht wird, wenn ein vorgegebener Leitwert der Spülflüssigkeit überschritten ist.

## Claims

1. Method for cleaning and thermal disinfection of objects in a cleaning and disinfection device, having the following steps:
• pre-cleaning (10),
• main cleaning (12) and
• final rinsing with thermal disinfection (14),
wherein the temperature of a rinsing liquid used in each case during each step (10, 12, 14) can be determined, and wherein
• the main cleaning (12) and the final rinsing (14a) are carried out with softened water or with fully desalinated water and
• the pre-cleaning (10) is exclusively carried out with softened water,
**characterized**
**in that** the pre-cleaning (10) is carried out at a temperature of 25 to 35 °C, wherein the temperature is kept constant during a time period of 1 s to 20 min.

2. Method according to claim 1, **characterized in that** it additionally comprises at least one of the subsequent steps:
• intermediate rinsing (11),
• neutralisation (13) or
• drying (15),
wherein each additional step can also be carried out several times.

3. Method according to claim 2, **characterized in that** the intermediate rinsing (11) is exclusively carried out with softened water.

4. Method according to claim 2 or 3, **characterized in that** the neutralisation (13) is carried out with softened water or with fully desalinated water.

5. Method according to any of claims 2 to 4, **characterized in that** the drying (15) is carried out at a temperature of 40 to 110 °C.

6. Method according any of claims 2 to 5, **characterized in that** the intermediate rinsing (11) and/or the neutralisation (13) are carried out under the supply of heat energy.

7. Method according to claim 6, **characterized in that** the duration of the intermediate rinsing (11) and/or the neutralisation (13) can be pre-defined.

8. Method according to claim 6 or 7 **characterized in that** the intermediate rinsing (11) and/or the neutralisation (13) can be carried out at a pre-defined temperature.

9. Method according to claim 5 or 8, **characterized in that** the temperature during the drying, the intermediate rinsing and/or the neutralisation is kept constant during a time period of 1 min to 60 min.

10. Method according to any of claims 2 to 9, **characterized in that** the step or the steps of the intermediate rinsing (11), the neutralisation (13) and/or the drying (15) last in each case at least until a temperature, which can be pre-defined, is reached.

11. Method according to any of the preceding claims, **characterized in that** besides the temperature at least another physical parameter, in particular the pH value and/or the conductance, of the rinsing liquid can be determined during each step.

12. Method according to claim 11, **characterized in that** the rinsing liquid is exchanged at the beginning of the final rinsing (14a) prior to the thermal disinfection (14b), if a pre-defined conductance of the rinsing liquid is exceeded.

## Revendications

1. Procédé pour le nettoyage et la désinfection thermique d'objets dans un appareil de nettoyage et de désinfection, comprenant les étapes suivantes:
* pré-nettoyage (10),
* nettoyage principal (12) et
* rinçage final avec désinfection thermique (14),
dans lequel la température d'un liquide de rinçage respectivement utilisé pendant chaque étape (10, 12, 14) peut être déterminée, et dans lequel
* le nettoyage principal (12) et le rinçage final (14a) sont effectués avec de l'eau adoucie ou de l'eau complètement dessalée et
* le pré-nettoyage (10) est effectué exclusivement avec de l'eau adoucie,
**caractérisé en ce que** l'on effectue le pré-nettoyage (10) à une température de 25 à 35°C, la température étant maintenue constante pendant un temps de 1 s à 20 min.

2. Procédé selon la revendication 1, **caractérisé en ce qu'**il comprend en plus au moins une des étapes suivantes:
* rinçage intermédiaire (11),
* neutralisation (13) ou
* séchage (15),
dans lequel chaque étape supplémentaire peut aussi être exécutée plusieurs fois.

3. Procédé selon la revendication 2, **caractérisé en ce que** l'on effectue le rinçage intermédiaire (11) exclusivement avec de l'eau adoucie.

4. Procédé selon la revendication 2 ou 3, **caractérisé en ce que** l'on effectue la neutralisation (13) avec de l'eau adoucie ou avec de l'eau complètement dessalée.

5. Procédé selon l'une quelconque des revendications 2 à 4, **caractérisé en ce que** l'on effectue le séchage (15) à une température de 40 à 110°C.

6. Procédé selon l'une quelconque des revendications 2 à 5, **caractérisé en ce que** l'on effectue le rinçage intermédiaire (11) et/ou la neutralisation (13) avec un apport d'énergie thermique.

7. Procédé selon la revendication 6, **caractérisé en ce que** la durée du rinçage intermédiaire (11) et/ou de la neutralisation (13) peut être prédéterminée.

8. Procédé selon la revendication 6 ou 7, **caractérisé en ce que** l'on effectue le rinçage intermédiaire (11) et/ou la neutralisation (13) à une température pouvant être prédéterminée.

9. Procédé selon la revendication 5 ou 8, **caractérisé en ce que** la température pendant le séchage, le rinçage intermédiaire et/ou la neutralisation est maintenue constante pendant un temps de 1 min à 60 min.

10. Procédé selon l'une quelconque des revendications 2 à 9, **caractérisé en ce que** l'étape ou les étapes de rinçage intermédiaire (11), de neutralisation (13) et/ou de séchage (15) durent respectivement au moins jusqu'à ce que l'on atteigne une température pouvant être prédéterminée.

11. Procédé selon l'une quelconque des revendications précédentes, **caractérisé en ce qu'**en plus de la température, au moins un autre paramètre physique, en particulier la valeur du pH et/ou la conductance, du liquide de rinçage peut être déterminée pendant chaque étape.

12. Procédé selon la revendication 11, **caractérisé en ce que** l'on échange le liquide de rinçage au commencement du rinçage final (14a) avant la désinfection thermique (14b), lorsqu'une conductance prédéterminée du liquide de rinçage est dépassée.
